# EUROPEAN PATENT APPLICATION

(11) **EP 3 296 019 A1**
(43) Date of publication of application: **21.03.2018**
(21) Application number: 16791936.4
(22) Date of filing: 22.03.2016
(51) Int. Cl.: B01L 3/00, G01N 21/01, B29C 65/48

(54) **CHIP USED FOR SAMPLE DETECTION AND PACKAGING METHOD THEREFOR**

(30) Priority: 12.05.2015 CN 201510239917
(71) Applicant: Tianjin Mnchip Technologies Co. Ltd., Tianjin 300457 (CN); Mnchip (Beijing) Technologies Co., Ltd., Beijing 100190 (CN)
(72) Inventor: WANG, Zhanhui, Tianjin 300457 (CN); CHEN, Tan, Tianjin 300457 (CN); CHEN, Fanglu, Tianjin 300457 (CN)
(74) Representative: ABG Patentes, S.L.
(86) International application number: PCT/CN2016/076986
(87) International publication number: WO 2016/180086

(57) **Abstract**

The present invention relates to a chip for sample detection and a packaging method of a chip, which can improve light transmittance of the chip and make the results of sample detection more accurate by disposing light-transmitting holes in an adhesive layer for sealing. The chip for sample detection includes a substrate and a covering plate, the substrate and the covering plate are sealed by an adhesive layer located therebetween, light-transmitting holes are disposed in the adhesive layer, detection grooves are disposed in the substrate, and the light-transmitting holes correspond to the detection grooves.

## Description

### TECHNICAL FIELD

The present invention relates to the field of sample detection, particularly to a chip used for sample detection and a packaging method therefor.

### BACKGROUND

Microfluidic chip technology is a new technique for precisely manipulating and controlling nanoliter and picoliter magnitude fluid (biological sample fluid) in micro-scale flow channels, which can integrate or substantially integrate basic operating units used in the chemical and biological fields and the like, such as sample preparation, reaction, separation, detection and cell culture, sorting, lysis, etc., into a chip having a size of several square centimeters (or even smaller).

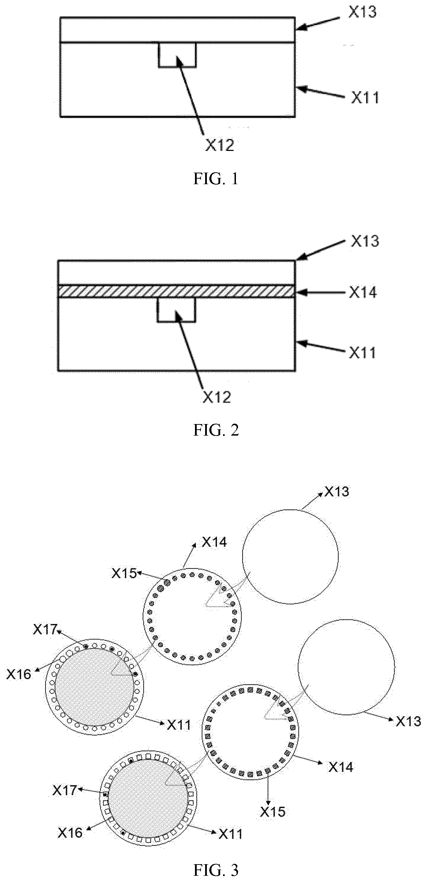

At present, packaging methods of a microfluidic chip are mainly divided into two kinds, one is a direct packaging method, and the other is an indirect packaging method. The direct packaging method is a method of directly sealing by means of the characteristics of the materials of a chip, without other medium between a substrate and a covering plate, the examples of which include the heat sealing method, the laser welding method and the ultrasonic welding method. This method uses the properties of thermoplastic materials to make the contact surfaces of the plastic materials reach the molten state, by means of heating, ultrasonic vibration etc., then the two layers will be packaged together by curing. The microfluidic chip made by this method mainly comprises two parts. As shown in FIG. 1, a micro channel 12 is formed on a substrate 11, and the substrate 11 having the micro channel 12 is packaged with a covering plate 13, which makes the micro channel 12 be in a relatively sealed state. The indirect packaging method refers to sealing the substrate and the covering plate together through curing of an intermedia which is attached therebetween. Generally, the intermedia is an adhesive layer, including UV-Curing adhesive, pressure-sensitive adhesive etc. FIG. 2 shows a structure of a microfluidic chip sealed with the adhesive layer, and the adhesive layer 14 is attached between the substrate 11 having the micro channel 13 and the covering plate 12.

For example, Patent Publication CN102923643A discloses a microfluidic chip, in which a slide (i.e., the covering plate) coated with a layer of UV-Curing adhesive is clung to a chip body (i.e., the substrate). As another example, Patent PublicationCN103495440A discloses sealing a microfluidic chip through pressure-sensitive adhesive.

When the microfluidic chip shown in FIG.2 is applied in optical detection, the content of a certain test item in a sample is deduced by detecting the degree of absorption of light after the reaction of the sample to be detected and a reagent in a detection groove. This requires that material of the chip has a very small degree of absorption of light to reduce the influence of the material itself on the absorbance of the reagent as much as possible, thus a material with excellent transmittance is selected as the material of the chip generally. When sealing by the adhesive, an adhesive layer is often coated on the surface of the covering plate. Since the adhesive layer itself also has a certain absorption of light, especially for an ordinary adhesive layer which has more absorption of light (relative to the optical adhesive), the final result of the reagent detection will be influenced.

### SUMMARY

The first purpose of the present invention is to provide a chip for sample detection to solve the problem that when the existing chip is applied in the field of optical detection, the light transmittance of the chip is low because the material itself absorbs light, and then causes an error for the result of reagent detection.

The second purpose of the present invention is to provide a packaging method of a chip for sample detection to make a chip with high transmittance, which is simple in steps, easy in operation and excellent in packaging effect.

The third purpose of the present invention is to provide an application of the chip for sample detection in biomedical detection, environmental detection, food hygiene detection, pharmacy or chemical industry.

The first aspect of the present invention provides a chip for sample detection, which includes a substrate and a covering plate, the substrate and the covering plate are sealed by an adhesive layer located therebetween, the adhesive layer is provided with light-transmitting holes, the substrate is provided with detection grooves, and the light-transmitting holes correspond to the detection grooves. The shape of the detection grooves and the light-transmitting holes may be circular, oval, square, or another polygon, the specific shape may be selected by those skilled in the art according to actual demands, and it is not limited in the present invention.

The chip described in the present invention is preferably a microfluidic chip, and the substrate is provided with one or more micro channels.

In embodiments of the present invention, the shape of the chip is circular, oval, rectangle, square or another polygon. Preferably, the shape of the chip is circular, and the detection grooves are disposed along a circumference of the substrate. The light-transmitting holes are disposed along a circumference of the adhesive layer and correspond to the detection grooves in the substrate. More preferably, the detection grooves are disposed along the circumference equidistantly, and the distances between centers of the detection grooves and a center of the chip are equal. The shape and size of the detection grooves may be the same or different.

In the embodiments of the present invention, the adhesive layer may be UV-Curing adhesive, pressure-sensitive double-sided adhesive or optical grade double-sided adhesive. More preferably, the adhesive layer is pressure-sensitive double-sided adhesive.

Those skilled in the art may understand that, compared with other packaging solutions (heat sealing, welding, etc.), sealing the chip with the pressure-sensitive double-sided adhesive has main advantages including that the cost is very low, large-scale industrial production may be carried out, the process of packaging may be carried out at room temperature (heat sealing requires a higher temperature, which may affect the reagent inside the chip), and it does not need specialized equipment for the packaging and can improve the yield of the chip packaging.

Those skilled in the art may also understand that although optical grade double-sided adhesive itself has a very low absorption of light and may improve the overall light transmittance of a chip, but the processing cost is relatively high, and optical grade double-sided adhesive itself may affect the reaction of the reagent and the sample.

In the embodiments of the present invention, the material of the substrate and the covering plate may be silicon wafer, glass, quartz, or thermoplastic polymers. Silicon wafer has advantages of good heat dissipation, high strength, moderate price, high purity and corrosion resistance. The micro channel network may be etched on the glass by photolithography and etching techniques, and the glass has a certain strength as well as good heat dissipation, transmittance and insulation, which is suitable for sample analysis.

The thermoplastic polymers mainly include polyamide (PI), polymethyl methacrylate (PMMA), polycarbonate (PC), polyethylene terephthalate (PET), polyvinyl chloride (PVC), polystyrene (PS), etc. More preferably, the material of the substrate is polymethyl methacrylate.

In a preferred embodiment of the present invention, the material of the covering plate is thermoplastic polymers. More preferably, the material of the covering plate is optical grade polycarbonate. Those skilled in the art may understand that the material of the covering plate and the substrate may be the same or different.

In the embodiments of the present invention, the number of the light-transmitting holes and the detection grooves is 1-30 respectively. Preferably, the number of the light-transmitting holes and the detection grooves is 5-30 respectively. Those skilled in the art may understand that the number of the light-transmitting holes and the detection grooves may be set according to specific or actual requirements, and it can also be greater than 30, which is not limited in the present invention.

In the embodiments of the present invention, reagents for detecting samples are placed in the detection grooves. When the chip is applied in the field of optical detection, the content of a certain test item in a sample is deduced by detecting the degree of absorption of light after the reaction of the sample to be detected and a specific reagent in a detection groove. The reagents may be solid or liquid, and in a specific embodiment of the present invention, the reagents are solid globules.

In a specific embodiment of the present invention, the light transmittance of the chip at 340nm is 85%-90%.

The microfluidic chip of the present invention may be a layered structure, and the layered structure may be single-layer or multi-layer, including 1, 2, 3 or more layers. In a specific embodiment, the layered structure of the chip may employ the structure described in Patent PublicationCN201210046040.0 or CN201110416978.2.

The chip has a two-layer structure including an upper layer and a lower layer, and the upper layer is connected with the lower layer watertight. The upper layer is provided with one or more through holes for adding samples, and the lower layer includes a sample groove, a diluents groove, a quantitative groove, a mixing groove, a reaction detection groove preplaced a reaction reagent, and multiple groups of micro channels controlling fluid flow.

The one or more through holes in the upper layer of the chip are used for sample and diluent injection. The sample groove and the diluent groove in the lower layer of the chip are used for storing the sample and diluent, which flows into the reaction detection groove through the micro channel after being quantified by the quantitative groove and being mixed.

The upper layer and the lower layer of the chip may be attached together by bonding, ultrasonic welding or laser welding, and a freeze-dried reaction reagent is preplaced in the reaction detection groove.

Those skilled in the art may understand that the purpose of disposing the micro channels in the chip is to make the chip integrate sample pretreatment, quantitative transportation, reaction and detection, which does not need to be equipped a professional sample pretreatment device or a manipulator. The operation is simple, and the whole detection process is fully automated without professional operation. Many detection indexes may be gained by one detection, and the detection cycle is short. The size of the chip is small, which may develop a miniaturized, integrated and portable detection device by integrating with a miniaturized detection device and realize field sampling analysis.

In a preferred embodiment of the present invention, the number of the micro channels disposed in the substrate is 1-100. Those skilled in the art may understand that other numbers of the micro channels may be disposed in the substrate to meet different needs, and it is not limited in the present invention.

In a preferred embodiment of the present invention, the adhesive layer is also provided with one or more through holes corresponding to the micro channels, and/or the sample groove, and/or the diluents groove, and/or the quantitative groove and/or the mixing groove. Disposing the one or more through holes in the adhesive layer may eliminate the negative influence which the adhesive layer may bring on the reagent or the sample.

The second aspect of the present invention provides a packaging method of a microfluidic chip, including: cutting an adhesive layer and forming multiple light-transmitting holes in the adhesive layer; attaching the adhesive layer after the processing of cutting holes to a covering plate; cutting the covering plate attached with the adhesive layer into the same shape and size as an substrate; and packaging the covering plate attached with the adhesive layer and the substrate, wherein, the light-transmitting holes correspond to detection grooves in the substrate.

Preferably, the adhesive layer is UV-Curing adhesive, pressure-sensitive double-sided adhesive or optical grade double-sided adhesive.

Preferably, the material of the substrate and the covering plate is silicon wafer, glass, quartz, or thermoplastic polymers. The thermoplastic polymers include polyamide (PI), polymethyl methacrylate (PMMA), polycarbonate (PC), polyethylene terephthalate (PET), polyvinyl chloride (PVC), polystyrene (PS), etc.

More preferably, the material of the substrate is polymethyl methacrylate (PMMA), and the covering plate is made of optical grade polycarbonate (PC).

In a specific embodiment of the present invention, the method includes:
S101: cutting an adhesive layer made of pressure-sensitive double-sided adhesive and forming multiple light-transmitting holes in the adhesive layer by use of a stamping tool;
S102: attaching the pressure-sensitive double-sided adhesive after the processing of cutting holes to the covering plate of optical grade polycarbonate;
S103: cutting the covering plate attached with the pressure-sensitive double-sided adhesive into the same shape and size as the substrate with the stamping tool; and
S104: packaging the covering plate attached with the pressure-sensitive double-sided adhesive and the substrate, wherein, the light-transmitting holes correspond to the detection grooves in the substrate.

Those skilled in the art may understand that other cutting methods may be used, such as laser drilling, chemical etching, etc. Steps S101 and S103 may adopt the same cutting method, or different ones.

The third aspect of the present invention provides an application of the chip in biomedical detection, environmental detection, food hygiene detection, pharmacy and chemical industry.

Preferably, the biomedical detection includes detection of whole blood, plasma, urine, saliva, semen, spinal cord and amniotic fluid for human or animals, and detection indexes include alanine aminotransferase, aspartate aminotransferase, gamma-glutamyltransferase, alkaline phosphatase, total bilirubin, direct bilirubin, total protein, albumin, urea, creatinine, uric acid, glucose, total cholesterol, triglyceride, high density lipoprotein, low density lipoprotein, very low density lipoprotein, serum magnesium, serum potassium, serum sodium, serum chloride, serum calcium, serum phosphorus, serum iron, serum ammonia and carbon dioxide.

Preferably, the environmental detection includes water quality detection, and detection indexes include organics, heavy metal ions, and reagents of pesticide residue in water.

Preferably, the food hygiene detection includes detecting microorganisms, additives, pesticide residues, contaminants and proteins in food.

Preferably, the detection of the pharmacy and the chemical industry includes the detection of pharmaceutical ingredients and chemical products.

Beneficial effects of the present invention compared with the prior art include:
1. The microfluidic chip provided by the present invention, in which light-transmitting holes are formed in the adhesive layer for sealing between the covering plate and the substrate, and corresponding to the detection grooves in the substrate, may increase the light transmittance, eliminate the negative influence that the adhesive layer may bring on the reaction of the reagent and the sample, improve sensitivity and accuracy of optical detection and increase the application prospect of the chip in different fields.
2. The packaging method of the microfluidic chip provided by the present invention is simple in steps, easy in operation, and may package the chip in quantities. The chip prepared by the packaging method provided by the present invention has high transmittance and may improve the sensitivity and accuracy of optical detection.

### BRIEF DESCRIPTION OF DRAWINGS

In order to make the content of the present invention easier to be clearly understood, the present invention is further described in detail according to specific embodiments of the present invention and in conjunction with the accompanying of the drawings in which:
FIG. 1 is a schematic view illustrating a basic structure of a microfluidic chip according to the prior art;
FIG. 2 is a schematic view illustrating a microfluidic chip having an adhesive layer according to the prior art;
FIG. 3 is a schematic view illustrating a microfluidic chip according to an embodiment of the present invention;
FIG. 4 is a flow chart illustrating a packaging method of the microfluidic chip according to an embodiment of the present invention;
FIG. 5 is a sectional view illustrating structures corresponding to steps of a packaging method of the microfluidic chip according to an embodiment of the present invention;
FIG. 6 is a front view illustrating structures corresponding to steps of a packaging method of the microfluidic chip according to an embodiment of the present invention.

Descriptions of reference signs: X11-substrate; X 12-micro channel; X 13-covering plate; X 14-adhesive layer; X 15-light-transmitting hole; X 16-detectiongroove; X 17-reagent.

### DETAILED DESCRIPTION

In the following description, the technical scheme in the embodiments of the present invention will be described clearly and completely in conjunction with the accompanying drawings. Obviously, the described embodiments are only a part of the embodiments of the present invention, not all of embodiments. All other embodiments obtained by those skilled in the art based on the embodiments of the present invention without any creative work are within the scope of protection of the present invention.

FIG. 3 is a schematic view illustrating a microfluidic chip according to an embodiment of the present invention. As shown in FIG. 3, the chip includes a substrate X 11 and a covering plate X 13, the shape and size of the substrate X 11 and the covering plate X 13 are the same. The substrate X 11 and the covering plate X 13 are sealed by an adhesive layer X 14 located therebetween, light-transmitting holes X 15 are formed in the adhesive layer X 14, detection grooves X 16are formed in the substrate X 11, and the light-transmitting holes X11correspondto the detection grooves X 16.

In an embodiment of the present invention, the chip is a microfluidic chip, and a micro channel X 12 is disposed in the substrate X 11.

In embodiments of the present invention, the shape of the chip may be circular, rectangle, square, or another polygon. The chip is circular in the embodiment shown in FIG. 3, and the detection grooves X 16 are disposed along the circumference of the substrate X 11. In order to facilitate detection, the distances between the centers of the detection grooves X 16 and the center of the chip are equal. The light-transmitting holes X 15 are disposed along the circumference of the adhesive layer X 14 and correspond to the detection grooves X 16 of the substrate X 11.

In an embodiment of the present invention, the microfluidic chip may be a layered structure, and the layered structure may be single-layer or multi-layer, including 1, 2, 3 or more layers. In a specific embodiment, the layered structure of the chip may employ the structure described in Patent Publication CN201210046040.0 or CN201110416978.2. That is, the chip has a two-layer structure including an upper layer and a lower layer, and the upper layer and the lower layer are connected watertight. The upper layer is provided with one or more through holes for adding samples, and the lower layer includes a sample groove, a diluents groove, a quantitative groove, a mixing groove, a reaction detection groove preplaced a reaction reagent, and multiple groups of micro channels controlling the fluid flow. Here, the one or more through holes in the upper layer of the chip are used for sample and diluent injection, and the sample groove and the diluents groove in the lower layer of the chip are used for storing the sample and the diluent, which flow into the reaction detection groove through the micro channel after being quantified by the quantitative groove and being mixed. The upper layer and the lower layer of the chip may be attached together by bonding, ultrasonic welding or laser welding, and a freeze-dried reaction reagent is preplaced in the reaction detection groove. Those skilled in the art may modify the structure of the chip according to actual requirements, which is not limited in the present invention.

In an embodiment of the present invention, the adhesive layer X 14 is also provided with one or more through holes corresponding to the micro channels X 12, and/or the sample groove, and/or the diluents groove, and/or the quantitative groove and/or the mixing groove etc. Disposing the one or more through holes in the adhesive layer may eliminate the negative influence which the adhesive layer may bring on the reagent or the sample.

In embodiments of the present invention, the shape of the detection grooves may be circular, oval, square or another polygon (the shapes shown in FIG. 3 are circular and square), which those skilled in the art can choose according to actual requirements, and it is not limited in the present invention. In some cases, the detection grooves may be disposed in the micro channels, and their shapes can also be irregular.

In the embodiments of the present invention, the adhesive layer X 14 may be UV-Curing adhesive, pressure-sensitive double-sided adhesive or optical grade double-sided adhesive. In the embodiment shown in FIG. 3, the adhesive layer is pressure-sensitive double-sided adhesive. Sealing the chip with the pressure-sensitive double-sided adhesive has the advantages of low cost and low packaging temperature requirement, and it does not need specialized equipment for packaging and may improve the yield of the chip packaging. Optical grade double-sided adhesive has a high light transmittance, but its cost is also high. Those skilled in the art may select different adhesives according to requirements.

In the embodiments of the present invention, the material of the substrate X 11 may be silicon wafer, glass, quartz, metal, alloy or thermoplastic polymers. The silicon wafer has advantages of excellent heat dissipation, high strength, moderate price, high purity and corrosion resistance. The glass has been widely used in the production of microfluidic chip, the micro channel network may be etched on the glass by photolithography and etching techniques, and it has a certain strength as well as good heat dissipation, transmittance and insulation, which is very suitable for usual sample analysis.

The thermoplastic polymers mainly include polyamide (PI), polymethyl methacrylate (PMMA), polycarbonate (PC), polyethylene terephthalate (PET), etc. In the embodiment shown in FIG. 3, the material of the substrate is polymethyl methacrylate. Those skilled in the art may understand that the thermoplastic polymers have lower cost and processing cost than other materials like silicon wafer and glass, and they are suitable for large-scale industrial production.

In the embodiment shown in FIG. 3, the material of the covering plate X 13 is optical grade polycarbonate, which can further improve the light transmittance. Those skilled in the art may understand that the material of the covering plate may be the same as the substrate. In an embodiment of the present invention, the number of the light-transmitting holes X 15 and the detection grooves X 16 is 1-30 respectively. In a preferred embodiment of the present invention, the number of the light-transmitting holes X 15 and the detection grooves X 16 is 5-30 respectively. In the embodiment shown in FIG. 3, the number is 30.

Those skilled in the art may understand that the number of the light-transmitting holes X 15 and the detection grooves X 16 may be set according to specific or actual requirements, and it is not limited in the present invention. Those skilled in the art may also understand that the size or shape of multiple detection holes and detection grooves may be the same or different, which may be selected by those skilled in the art according to actual requirements, and it is not limited in the present invention.

In an embodiment of the present invention, reagents X 17 for detecting samples are placed in the detection grooves X 16. The reagents X 17 may be in a solid state or a liquid state, which is not limited in the present invention. In the embodiment shown in FIG. 3, the reagents X 17 are solid globules.

The present invention also provides a packaging method of a microfluidic chip. The method includes:
Step 1: cutting an adhesive layer and forming multiple light-transmitting holes in the adhesive layer;
Step 2: attaching the adhesive layer after the processing of cutting holes to a covering plate;
Step 3: cutting the covering plate attached with the adhesive layer into the same shape and size as a substrate; and
Step 4: packaging the covering plate attached with the adhesive layer and the substrate, wherein, the light-transmitting holes correspond to detection grooves in the substrate.

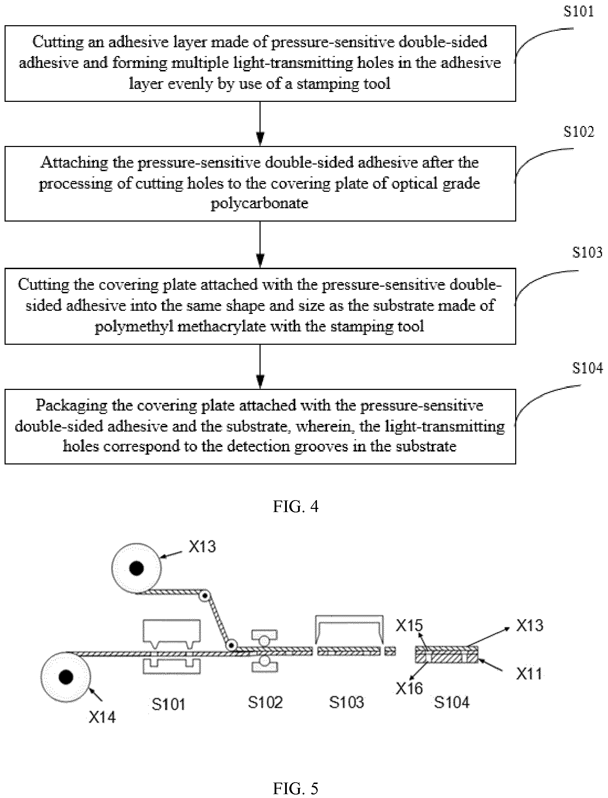

In a specific embodiment of the present invention, the material of the adhesive layer is pressure-sensitive double-sided adhesive, the material of the covering plate is optical grade polycarbonate, the material of the substrate is polymethyl methacrylate, the tool for cutting is a stamping tool, and the detailed flowchart of the method is shown in FIG. 4.

S101: cutting an adhesive layer made of pressure-sensitive double-sided adhesive and forming multiple light-transmitting holes in the adhesive layer evenly by use of a stamping tool;

S102: attaching the pressure-sensitive double-sided adhesive after the processing of cutting holes to the covering plate of optical grade polycarbonate;

S103: cutting the covering plate attached with the pressure-sensitive double-sided adhesive into the same shape and size as the substrate made of polymethyl methacrylate with the stamping tool; and

S104: packaging the covering plate attached with the pressure-sensitive double-sided adhesive and the substrate, wherein, the light-transmitting holes correspond to the detection grooves in the substrate.

Those skilled in the art may understand that the stamping tools used in steps S101 and S103 above may be the same or different, as long as they can be used. In addition, the method of cutting holes is not limited to using a stamping tool, other conventional methods such as laser drilling, chemical etching and the like can also be used, and those skilled in the art can choose one depending on actual requirements.

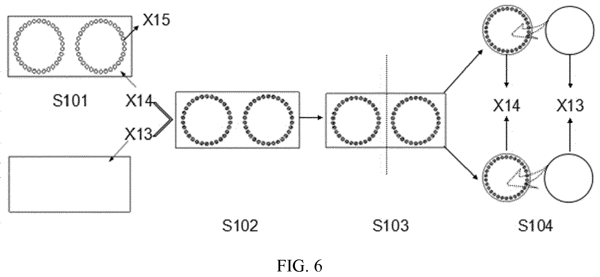

FIGs. 5 and 6 are a sectional view and a front view schematically illustrating structures corresponding to the above method respectively, and each step in FIGs. 5 and 6 corresponds to one of steps S101-S104 in FIG. 4 respectively.

In an embodiment of the present invention, the light transmittance of the chip in which the light-transmitting holes are not formed in the adhesive layer and in which the light-transmitting holes are formed in the adhesive layer are compared, and the optical grade polycarbonate (PC) is used for the light transmittance detection. The experimental instrument used in this experiment is Celecare M1, which is an automatic biochemical analyzer produced by Tianjin Mnc Technologies Co., Ltd, and the wavelength is 340nm. The chips to be tested were divided into two groups, group A comprised PC films in which the light-transmitting holes were not formed in the double-sided adhesive, and group B comprised PC films in which the light-transmitting holes were formed in the double-sided adhesive. Two groups of PC films attached with double-sided adhesive were fixed on a fixture and inserted into the instrument, and the detection of light exposure was carried out. The light intensity values before and after the light passed through the PC films were counted, thus the absorbance degrees were calculated. Each group included 10 pieces of test samples, and the test result is shown in Table 1.

**Table 1 Data Comparison of Transmittance Experiments**

| Group A | | | | Group B | | | |
|---|---|---|---|---|---|---|---|
| Wavelength 340nm | | | | | | | |
| Incident light intensity I₀ | Transmitted light intensity I | Transmittance T | Absorbance degree A0 | Incident light intensity I0 | Transmitted light intensity I | Transmittance T | Absorbance degree A0 |
| 13652 | 9561 | 70.03% | 0.154693 | 13669 | 12413 | 90.81% | 0.0418599 |
| 13673 | 9421 | 68.90% | 0.1617668 | 13546 | 12356 | 91.22% | 0.0399332 |
| 13668 | 9652 | 70.62% | 0.1510877 | 13742 | 12525 | 91.14% | 0.0402722 |
| 13659 | 9498 | 69.54% | 0.1577867 | 13581 | 12425 | 91.49% | 0.0386354 |
| 13682 | 9521 | 69.59% | 0.157467 | 13687 | 12548 | 91.68% | 0.0377338 |
| 13661 | 9621 | 70.43% | 0.1522623 | 13671 | 12523 | 91.60% | 0.0380919 |
| 13678 | 9542 | 69.76% | 0.1563832 | 13681 | 12422 | 90.80% | 0.0419263 |
| 13675 | 9487 | 69.37% | 0.1587984 | 13662 | 12358 | 90.46% | 0.0435661 |
| 13653 | 9671 | 70.83% | 0.1497567 | 13658 | 12421 | 90.94% | 0.0412305 |
| 13666 | 9703 | 71.00% | 0.1487354 | 13649 | 12365 | 90.59% | 0.0429067 |

Through the above comparison experiments it is found that the light transmittance of the chip without the light-transmitting holes is only about 70% in 340nm band, while the light transmittance of the chip after the processing of cutting adhesive may be up to around 89% in 340nm band, which eliminates the influence of the absorbance of the adhesive layer itself. The chip prepared by the packaging method provided by the present invention can not only improve the light transmittance of the chip itself, but also improve the consistency of the light transmittance of the detection locations of the chip after the processing of cutting adhesive. Since solid reagents are preplaced in the detection grooves, the processing of cutting adhesive may also eliminate the influence of the adhesive layer itself on the reagents reaction.

The chip according to the present invention has good application prospects in biomedical detection, environmental detection, food hygiene detection, pharmacy and chemical industry. The chip can be used for field assay in the fixed sites or fields, detected by traditional biochemical analyzer or portable biochemical analyzer, and can be analyzed with multiple samples and multiple indexes simultaneously. The biomedical detection includes detection of whole blood, plasma, urine, saliva, semen, spinal cord, and amniotic fluid for human or animals, and detection indexes include alanine aminotransferase (ALT), aspartate aminotransferase (AST), gamma-glutamyltransferase (γ-GT), alkaline phosphatase (ALP), total bilirubin (TBIL), direct bilirubin (DBIt), total protein (TP), albumin (Alb), urea (Urea), creatinine (Cr), uric acid (UA), glucose (Glu), total cholesterol (TC), triglyceride (TG), high density lipoprotein (HDL), low density lipoprotein (VLDL), very low density lipoprotein (LDL), serum magnesium (Mg), serum potassium (K), serum sodium (Na), serum chloride (Cl), serum calcium (Ca), serum phosphorus (P), serum iron (Fe), serum ammonia (NH)and carbon dioxide (CO₂).

The environmental detection includes water quality detection, and detection indexes include organics, heavy metal ions, and reagents of pesticide residue in water.

The food hygiene detection includes detecting microorganisms, additives, pesticide residues, contaminants and proteins in food.

The detection of the pharmacy and the chemical industry includes the detection of pharmaceutical ingredients and chemical products.

The above embodiments are only to illustrate the technical conception and characteristics of the present invention, and not to limit the present invention. Any modification and equivalent replacement etc., made within the spirit and principles of the present invention, shall be included in the protection scope of the present invention.

## Claims

1. A chip for sample detection, comprising a substrate and a covering plate, the substrate and the covering plate being sealed by an adhesive layer located therebetween, light-transmitting holes are disposed in the adhesive layer, detection grooves are disposed in the substrate, and the light-transmitting holes correspond to the detection grooves.

2. The chip for sample detection according to claim 1, wherein the chip is a microfluidic chip, and one or more micro channels are disposed in the substrate.

3. The chip for sample detection according to claim 2, wherein the shape of the chip is circular, the detection grooves are disposed along a circumference of the substrate, and the light-transmitting holes are disposed along a circumference of the adhesive layer and corresponding to the detection grooves of the substrate.

4. The chip for sample detection according to claim 1, wherein the adhesive layer is UV-Curing adhesive, pressure-sensitive double-sided adhesive or optical grade double-sided adhesive.

5. The chip for sample detection according to claim 1, wherein the material of the substrate and/or the covering plate is silicon wafer, glass, quartz, metal, alloy or thermoplastic polymers.

6. The chip for sample detection according to claim 5, wherein the thermoplastic polymers include polyamide, polymethyl methacrylate, polycarbonate, polyethylene terephthalate, polyvinyl chloride and polystyrene.

7. The chip for sample detection according to claim 5, wherein the material of the covering plate is optical grade polycarbonate.

8. The chip for sample detection according to any one of claims 1-7, wherein the detection grooves are provided with reagents for detecting samples, and the reagents are in a solid state or a liquid state.

9. A packaging method of a chip for sample detection, comprising:
cutting an adhesive layer and forming multiple light-transmitting holes in the adhesive layer;
attaching the adhesive layer after the processing of cutting holes to a covering plate;
cutting the covering plate attached with the adhesive layer into the same shape and size as a substrate; and
packaging the covering plate attached with the adhesive layer and the substrate, wherein the light-transmitting holes correspond to detection grooves in the substrate.

10. The packaging method of the chip for sample detection according to claim 9, comprising:
cutting an adhesive layer made of pressure-sensitive double-sided adhesive and forming multiple light-transmitting holes in the adhesive layer evenly by use of a stamping tool;
attaching the pressure-sensitive double-sided adhesive after the processing of cutting holes to the covering plate of optical grade polycarbonate;
cutting the covering plate attached with the pressure-sensitive double-sided adhesive into the same shape and size as the substrate made of polymethyl methacrylate with a stamping tool; and
packaging the covering plate attached with the pressure-sensitive double-sided adhesive and the substrate, wherein the light-transmitting holes correspond to the detection grooves in the substrate.

11. The application of the chip for sample detection according to any one of claims 1-8 in biomedical detection, environmental detection, food hygiene detection, pharmacy and chemical industry.
